(19) Europäisches Patentamt
European Patent Office
Office européen des brevets

(11) EP 0 908 455 B1

(12) EUROPÄISCHE PATENTSCHRIFT

(45) Veröffentlichungstag und Bekanntmachung des Hinweises auf die Patenterteilung:
**10.07.2002 Patentblatt 2002/28**

(51) Int Cl.[7]: **C07D 313/00**, C11B 9/00

(21) Anmeldenummer: **98118789.1**

(22) Anmeldetag: **05.10.1998**

(54) **Macrocyclen**

Macrocycles

Macrocycles

(84) Benannte Vertragsstaaten:
**CH DE ES FR GB IT LI NL**

(30) Priorität: **09.10.1997 CH 236297**

(43) Veröffentlichungstag der Anmeldung:
**14.04.1999 Patentblatt 1999/15**

(73) Patentinhaber: **Givaudan SA**
**1214 Vernier-Genève (CH)**

(72) Erfinder:
- **Frater, Georg**
  **8400 Winterthur (CH)**
- **Helmlinger, Daniel**
  **8600 Dübendorf (CH)**
- **Mueller, Urs**
  **8051 Zürich (CH)**

(74) Vertreter: **Patentanwälte**
**Schaad, Balass, Menzl & Partner AG**
**Dufourstrasse 101**
**Postfach**
**8034 Zürich (CH)**

(56) Entgegenhaltungen:
**EP-A- 0 424 787** **EP-A- 0 818 452**
**WO-A-97/32948** **CA-A- 1 221 105**
**US-A- 2 234 551** **US-A- 3 681 395**
**US-A- 4 541 950**

- **CHEMICAL ABSTRACTS, vol. 96, no. 27, 1982 Columbus, Ohio, US; abstract no. 6612e, Seite 599; XP002091173 & JP 08 190076 A (TORAY INDUSTRIES)**
- **P. Kraft, W. Tochtermann, Liebigs Ann. Chem., 1994, 1161-1164**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft Riechstoffkompositionen mit einem Gehalt an Macrocyclen, nämlich an 13-Methyl-oxacyclopentadec-10-en-2-on (Verbindung I).

13-Methyl-oxacyclopentadec-10-en-2-one soll sämtliche möglichen Isomeren umfassen:

Racemate und die optisch aktiven Verbindungen, das heisst die R- und S-Formen, cis/trans-Verhältnis

Kerschbaum isolierte 1927 Ambrettolid (Z-Oxacycloheptadec-8-en-2-on) aus Ambrettesamenöl (Moschuskörneröl) (Ber. 60,902,(1927). Aus demselben Öl isolierte Maurer Z-Oxacyclopentadec-6-en-2-on (B. Maurer, A. Grieder, Helv. Chim. Acta, 60, 1155, (1977). Die Firma IFF schützt sich in einem Patent die Synthese von E-Oxacycloheptadec-10-en-2-on (USP 4 064 144).

Mookherjee beschreibt die Synthese eines Gemischs von Oxacycloheptadec-8-en-2-on, Oxacycloheptadec-9-en-2-on, Oxacycloheptadec-10-en-2-on, Oxacycloheptadec-11-en-2-on, wobei die dritte Verbindung zu 80 % im oben erwähnten Gemisch vorhanden ist. Eine Stereochemie der Verbindungen wird nicht angegeben. (B.D. Mookherjee, R. W. Trenkle, R.R. Patel, J. Org. Chem. 37, 24, 3846, (1972)). Bei der oben erwähnten Synthese handelt es sich um eine Pyrolyse eines Esters, eine selektive Bildung eines cis-Olefins ist nicht zu erwarten (siehe dazu Houben-Weyl Band V /1b,105, 1972).

Firmenich liess sich ein Gemisch von hauptsächlich E-Oxacyclohexadec-12-en-2-on und E-Oxacyclohexadec-13-en-2-on schützen, wobei die Z-Isomeren nur in geringem Prozentsatz vorhanden sind (EP 0424 787).

Der Firma IFF ist die Anwendung von Oxacyclohexadec-11-en-2-on geschützt (USP 4 541 950), wobei das cis/trans-Verhältnis des erhaltenen Produkts nicht angegeben ist. Da aber das Produkt durch Metathese erhalten wurde, ist davon auszugehen, dass der trans-Anteil höher ist als der cis-Anteil, siehe dazu A. Fürstner, K. Langemann, J. Org. Chem. 61, 3942, 1996.

Die französische Firma Mane et Fils in F-06620 Bar s/Loup verkauft eine Verbindung unter dem Namen Cis-iso-ambrettolide. Es handelt sich dabei um Z-Oxacycloheptadec-11-en-2-on (Parfums, Cosmétiques, Actualiées, No 128, avril/mai 63, 1996).

C. Collaud (Helv. chim. acta 25, 965, 1942) beschreibt die Herstellung eines Gemischs von Oxacycloheptadec-6-en-2-on und Oxacycloheptadec-7-en-2-on, wobei die Stereochemie der Produkte nicht angegeben wird. Bei der oben erwähnten Synthese handelt es sich wiederum um eine Pyrolyse eines Esters, eine selektive Bildung eines cis-Olefins ist nicht zu erwarten.

Neben extrem niedrigen Schwellenwerten besitzen die Verbindung I und ihre Isomere eine sehr gute Haftfestigkeit. Alle weisen intensive Moschusnoten auf, die oft auch von pudrigen, fruchtigen, blumigen Nebennoten begleitet sind. Die Verbindungen weisen auch ambraähnliche, erdige nach Ambrettesamen riechende Nebenoten aus. Ein besonders schönen parfümistischen Effekt weist bei dominanter Moschusnote Z-13-Methyl-oxacyclopentadec-10-en-2-on auf. Die Moschusnote wird durch eine pudrige, fruchtige Komponente abgerundet.

Nachfolgend werden bevorzugte erfindungsgemässe Verbindungen näher charakterisiert:

- Z-13-Methyl-oxacyclopentadec-10-en-2-on:

  Diese Verbindung zeichnet sich durch einen sehr niedrigen Schwellenwert aus. Der Olfaktometerschwellenwert ist 0,578 ng/l. Der GC-Schwellenwert 0,1 ng/l. Dies ergibt einen "Geruchswert" ( = odour value) von 18038 [siehe N. Neuner-Jehle, F. Etzweiler, in Perfumes: Art Science and Technology, Edited by P.M. Müller, D. Lamparsky]. Dies ist der höchste "Geruchswert" der für einen macrocyclischen Moschus bekannt geworden ist. Zum Beispiel hat Musk R1 (1,7-Dioxa-cycloheptadecan-8-on) einen Schwellenwert von 0,33 ng/l; einen Dampfdruck von 3,24 µg/l und einen "Odour value" von 3074. Thibetolid hat einen Schwellenwert von 2,16 ng/l, einen Dampfdruck von 6,64 µg/l und einen "Odour value" von 3074. Alle andern macrocyclischen Moschusriechstoffe haben noch geringere Werte. Z-13-Methyl-oxacyclopentadec-10-en-2-on zeichnet sich durch einen pudrigen, moschusartigen, laktonartigen, fruchtigen Geruch aus;

Die Verbindung I und ihre Isomere lassen sich generell wie die bekannten Moschusriechstoffe einsetzen, sie harmonisieren also mit einer Vielzahl natürlicher wie synthetischer Produkte, die häufig in Riechstoffkompositionen eingesetzt werden. Insbesondere in der Basisnote erzielen sie in Kombination mit holzigen und ambrierten Akkorden, Patchouliöl, Cedern- und Sandelholzriechstoffen, interessante Effekte. Blumige Herz(Mittel)noten verleihen den Verbindungen Eleganz und Strahlungskraft. Einige Beispiele für besonders gut harmonisierende Stoffklassen sind:

- Naturprodukte, wie Eichenmoos Absolue, Geraniumöl, Jasmin Absolue, Patchouliöl, Rosenöl, Sandelholzöl, Vetiverol und Ylang-Ylang-Öl, etc.

- Alkohole, wie Citronellol, Ebanol®, Geraniol, Linalool, Phenylethylalkohol und Sandalore®, etc.;

- Aldehyde und Ketone, wie Florozone® (3-(4-Ethylphenyl)-2,2-dimethylpropional), Hydroxycitronellal, Iso-E-Super® (1,2,3,4,5,6,7,8-Octahydro-2,3,8,8-tetramethal-2-octanaphthalin), Isoraldein®, Maltol, Methyl-, cedrylketon, Methyljonon und Vanillin, etc.;

- Ether und Acetale, wie Ambrox, Geranylmethylether, Rosenoxid und Spirambrene® (2',2',3,7,7-Pentamethyl-spiro [bicyclo[4.1.0]heptan-2,5'-[1,3]dioxan]), etc.;

- Ester und Lactone, wie Berryflor®, γ-Decalacton und γ-Undecalacton, etc. .

Ihre Vielseitigkeit ermöglicht einen breiten Einsatz der Verbindung I und ihrer Isomeren nicht nur in den süssen orientalischen Kreationen, sondern auch in den Duftrichtungen 'Fougere', 'Chypre' und 'Floral'. Durch die niedrigen Schwellenwerte und die gute Haftfestigkeit werden neben der Luxusparfümerie auch Kompositionen für kosmetische Produkte, Waschmittel und ähnliche Massenerzeugnisse erschlossen.

Die Verbindungen I und ihre Isomere lassen sich in weiten Grenzen einsetzen, die beispielweise von ca. 0.1 (Detergenzien) - ca. 40 Gew.-% (alkoholische Lösungen) in Kompositionen reichen können, ohne dass diese Werte jedoch Grenzwerte darstellen sollen, da der erfahrene Parfümeur auch mit noch geringeren Konzentrationen Effekte erzielen oder aber mit noch höheren Dosierungen neuartige Komplexe aufbauen kann. Die bevorzugten Konzentrationen bewegen sich zwischen ca. 3 und ca. 20 %. Die mit Verbindung I und ihrer Isomeren hergestellten Kompositionen lassen sich für alle Arten von parfümistischen Verbrauchsgütern einsetzen (Eaux de Cologne, Eaux de Toilette, Extraits, Lotionen, Crèmes, Shampoos, Seifen, Salben, Puder, Desodorantien, Detergenzien, etc.).

Die Verbindung I und ihre Isomere können demgemäss bei der Herstellung von Kompositionen und -wie obige Zusammenstellung zeigt- unter Verwendung einer breiten Palette bekannter Riechstoffe bzw. Riechstoffgemische verwendet werden. Bei der Herstellung solcher Kompositionen können die oben angeführten Riechstoffe bzw. Riechstoffgemische nach dem Parfümeur bekannter Art und Weise verwendet werden, wie z.B. aus W.A. Poucher, Perfumes, Cosmetics, Soaps, 2. Bd., 7, Aufl. Chapman und Hall, London 1974, hervorgeht.

Das Verfahren zur Herstellung der Verbindungen bzw. Gemische I ist dadurch gekennzeichnet, dass man eine Verbindung der Formel

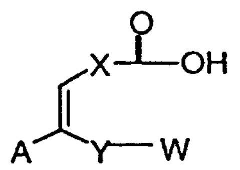

II

worin A = H, X = $-(CH_2)_7-$ und Y = $-CH_2-CH(CH_3)-(CH_2)_2-$ darstellen, und W OH, Alkanoat, zum Beispiel Acetat, oder eine Abgangsgruppe darstellt, wie Mesylat, Tosylat, I, Br, Cl usw., in an sich bekannter Weise, nämlich bei erhöhter Temperatur und unter basischen Bedingungen, laktonisiert.

Die Verbindung II kann dadurch erhalten werden, dass man eine Verbindung der Formel

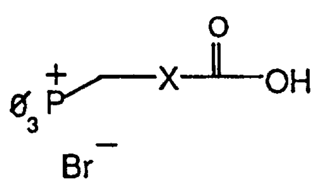

III

mit einer Verbindung der Formel

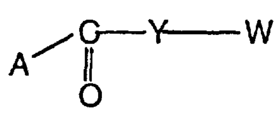

IV

einer Wittig-Reaktion unterwirft.

Die Laktonisierung einer Verbindung der Formel II zu einer Verbindung der Formel I kann beispielsweise nach der Methode von Collaud (BP No. 490 044, 4. Januar 1937, C. Collaud, Helv. Chim. Acta, 965, 1942) erfolgen.

Hier erfolgt zuerst die Herstellung eines ω-Hydroxycarbonsäure-2,3-dihydroxy-propylesters durch Behandlung des Natriumsalzes der entsprechenden ω-Hydroxycarbonsäure II mit Chlorpropan-1,2-diol, gefolgt von einer internen Umesterung in Gegenwart eines Methanolats, insbesondere Natriummethanolat. Das entstandene Monomer wird in Gegenwart eines hochsiedenden Lösungs- bzw. Schleppmittels, zum Beispiel Glycerin, aus dem Reaktionsgemisch herausdestilliert.

Eine zweite Möglichkeit zur Laktonisierung der Verbindung der Formel II zu einer Verbindung der Formel I ist beispielsweise im Patent CH 344 712 beschrieben. Die ω-Hydroxycarbonsäure wird bei hoher Temperatur in Gegenwart einer Base (KOH, NaOH, usw.) und Glycerin erhitzt, wobei ein Polyester entsteht. Nachträglich erfolgt eine interne Umesterung in Gegenwart von Natriummethylat. Das entstandene Monomer wird in einem hochsiedenden Lösungsmittel, zum Beispiel Glycerin, aus dem Reaktor herausdestilliert.

Wenn W gleich I, Br oder Cl ist, kann die Laktonisierung von Verbindung der Formel II zu einer Verbindung der Formel I auch nach der Methodik von Mandolini et al. erfolgen (G. Galli, G. Giannelli, G. Illuminati, L. Mandolini, J. Org. Chem., Vol. 44, No. 8, 1258, 1979). Das ω-Bromoalkanoat II wird hier zu einer gut gerührten Suspension von Kaliumcarbonat in Dimethylsulfoxyd zugetropft.

Die Anwendung dieser bekannten Methode ist aber tel quel zur Herstellung von Riechstoffen nicht geeignet, da sich schwer zu entfernende Schwefelnoten im Produkt bilden. Es wurde nun gefunden, dass Dimethylsulfoxyd überraschend durch ein schwefelfreies Lösungsmittel, insbesondere N-Methylpyrrolidon ersetzt werden kann und auf diese Art ein geruchlich einwandfreies Produkt erhalten wird.

Die Herstellung der Verbindung II kann nach den an sich bekannten Methoden der Wittig-Reaktion erfolgen. Es wird somit ein Phosponiumsalz der Formel III zweckmässigerweise zunächst mit (etwa zwei Aequivalenten) einer starken Base behandelt, wobei ein Ylid entsteht. Als Basen dienen zum Beispiel Kalium-t-butylat, Kalium-t-amylat, Methyllithium, Butyllithium, Phenyllithium, Kaliumhydrid, Natriumhydrid, Hexamethyldisilazan (als Kalium oder Natriumsalz), Lithiumdiisopropylamid usw. Als Medium für die weitere Umsetzung mit der Verbindung IV dient vorzugsweise ein aprotisches Lösungsmittel wie Diethylether, Tetrahydrofuran, Benzol, Toluol, Hexan, Dimethylformamid, HMPA (Hexamethalphosphorsäuretriamid) usw. Die Temperaturen sind nicht kritisch, und der zweckmässige Temperaturbereich kann breit sein (ca. - 78 bis ca. 100 °C).

Zwar wurde die Wittig-Reaktion sporadisch zur Synthese von Vorstufen von macrocyclischen Verbindungen angewandt (Nicolaou et al., J. Org. Chem. 44, 4011, 1979). Büchi und Wuest (Helv. Chim. Acta, 62, 2661, 1979) haben die eine Wittig-Reaktion einschliessende Horner-Emmons-Kondensation zur Synthese der zyklischen $C_{15}$-Ketone Exaltone und Muscone angewandt. Aber die Verwendbarkeit zur Synthese von macrocyclischen Laktonen, zudem verschiedener Ringgrössen, ferner auch mit Doppelbindungen in verschiedenen Lagen, ist bis jetzt nicht bekannt geworden.

Es ist erst das erfindungsgemässe Verfahren, dass die Herstellung von einer Verbindung I, die hauptsächlich in der cis-Form vorliegt, erlaubt.

Beispiel 1

100 g 3-Methyl-1,5-pentandiol (0.846 Mol), 108 ml Essigsäureethylester (1.1 Mol), 21 g Amberlyst® 15 gelöst in 420 ml Toluol wurden 3 Stunden bei 94 °C auf Rückflusstemperatur erhitzt. Dann liess man abkühlen, filtrierte über Celite und engte am Rotationsverdampfer ein. Man erhielt 104.5 g Rohprodukt als Gemisch aus 3-Methyl-1,5-pentandiol (25 %), 3-Methyl-1,5-pentandiolmonoacetat (50 %) und 3-Methyl-1,5-pentandioldiacetat (25 %).

Beispiel 2

189 g Rohprodukt (aus dem vorhergehendem Ansatz) und 9 g Kaliumbromid (76 mMol) wurden in 377 ml Methylenchlorid vorgelegt und bei -10 °C mit 1.18 g 2,6,6-Tetramethylpiperidin-1-oxyl-Radikal (7.55 mMol) versetzt. Bei 0 - 5 °C wurden 780 ml 12 - 15 proz. Hypochloritlösung (mit 19 g/l $NaHCO_3$ auf pH 9 gestellt) innerhalb von 50 Minuten zugetropft. Nach 40 Minuten stieg die Temperatur trotz Kühlung auf 18 °C. Man liess 1 Stunde bei 2 - 8 °C nachrühren. Anschliessend wurden nochmals 195 ml 12 - 15 proz. Hypochloritlösung (mit 19 g/l $NaHCO_3$ auf pH 9 gestellt) innerhalb von 15 Minuten bei 0 - 5 °C zugetropft. Man liess 30 Minuten nachrühren, wobei die Temp. auf 10 °C stieg. Es wurden nochmals 195 ml 12- 15 proz. Hypochloritlösung (mit 19 g/l $NaHCO_3$ auf pH 9 gestellt) innerhalb von 10 Minuten zugetropft, wobei die Temp. auf 6 °C stieg. Man liess 1 Stunde und 30 Minuten bei 0 - 5 °C nachrühren. Das Reaktionsgemisch wurde auf Wasser gegossen und zweimal mit Methylenchlorid extrahiert. Die organische Phase wurde mit 2N HCl + 5 g KI und mit Wasser + 9 g $Na_2S_2O_3 \cdot 5H_2O$ gewaschen (Peroxidtest negativ). Danach wurde über $Na_2SO_4$ getrocknet und eingeengt. Man erhielt 177 g Rohprodukt. Dieses Rohprodukt wurde destilliert und ergab 114

g Produkt (Sdp 75 - 76 °C, 0.2 bar) (Gehalt: -70 % 3-Methyl-5-oxopentanolacetat nach $^1$H-N MR).

Beispiel 3

156 g (8-Carboxyoctyl)-triphenylphosphoniumbromid (0.312 Mol) wurden pulverisiert und in 330 ml Tetrahydrofuran vorgelegt. Nach 10 Minuten Rühren wurde auf - 20 °C abgekühlt und mit 69.9 g Kalium-*t*-butylat (0.622 Mol) in 90 ml Tetrahydrofuran zügig versetzt. Die Temp. stieg auf 6 °C und das Reaktionsgemisch verfärbte sich dunkelrot. Man verdünnte mit 50 ml Tetrahydrofuran und liess 1 Stunde bei 5 - 10 °C nachrühren. Dann wurde auf -20 °C abgekühlt und mit 70 g (0.30 Mol, 70 %) 3-Methyl-5-oxopentanolacetat in 50 ml Tetrahydrofuran versetzt. Die Temp. stieg dabei auf 15 °C. Man liess 1 Stunde bei -10 °C bis -8 °C rühren und danach 1 Stunde bei Raumtemp. Man erwärmte auf 35 °C und rührte weitere 30 Minuten. Das Reaktionsgemisch wurde auf Wasser gegossen, mit 2N NaOH auf pH 12 gestellt und zweimal mit Ether extrahiert. Die organische Phase wurde mit 2N NaOH gewaschen, die wässrige Phase wurde mit 85 prozentige ortho-Phosphorsäure angesäuert und zweimal mit Ether extrahiert. Die organische Phase wurde mit gesättigter Kochsalzlösung gewaschen, über Natriumsulfat getrocknet und eingeengt. Man erhielt 106 g Rohprodukt, das chromatographisch gereinigt wurde. Bei den Verunreinigungen (~36 g) handelt es sich hauptsächlich um Triphenylphosphinoxyd. Das Produkt besteht aus einem Gemisch von 9*Z*-14-Acetoxy-12-methyltetradec-9-ensäure und 9*Z*-14-Hydroxy-12-methyltetradec-9-ensäure im Verhältnis 2:1.

Spektren von 9*Z*-14-Hydroxy-12-methyltetradec-9-ensäure *Z*/*E* = 94/6.

IR(Film): 3336; 3005; 2928; 2855; 1711; 1458; 1246; 1057.

$^1$H-NMR ($CDCl_3$, 200 $MH_3$) 5.4 (2H) m; 3.7 (2H) m; 2.32 (2H) t *J* = 7 Hz; 0.9 (3H) d *J* = 6.2.

MS 238 (2); 150 (4); 136 (5); 109 (16); 95 (32); 81 (100); 67 (48); 55 (76); 41 (48).

Beispiel 4

Apparatur:

350 ml 3-Hals-Sulfierkolben, Spezialaufsatz (siehe Fig. 1), Rückflusskühler, Thermostat, 2 Kühlfallen, HV-Pumpe mit Vakuumkonstanthalter.

In einem 350 ml Sulfierkolben mit Destillationsaufsatz wurden 53.5 g Rohprodukt (~ 65 %) des vorhergehenden Ansatzes, 230 ml Glycerin und 1 g Kaliumhydroxid (85 %) in 1 ml Wasser vorgelegt. Im Hochvakuum wurde das gebildete Wasser, Essigsäure und 4 - 5 ml Glycerin abdestilliert. Darauf wurde der Destillationsaufsatz entfernt und der Kolben mit dem beschriebenen Spezialaufsatz ausgerüstet. Dieser wurde mit 150 ml Glycerin gefüllt. Man liess abkühlen und gab 1 g Natriummethylat zu. Das gebildete Methanol wurde im Vakuum entfernt. Im Hochvakuum. (3 -4 mbar) wurde 18 Stunden auf 170- 180°C unter Rückfluss (Innenanfangstemp. 155 °C) erhitzt. Nach dieser Zeit hatte sich eine 15 mm dicke Schicht Lacton im Spezialaufsatz gebildet. Man liess abkühlen, gab 1 g Natriummethylat zu und destillierte das gebildete Methanol im Vakuum ab. Nach 42 stündiger Destillation wiederholte man die Zugabe. Nach insgesamt 66 stündigem Rückfluss liess man abkühlen. Der Inhalt des Aufsatzes wurde mit Wasser verdünnt und viermal mit Ether extrahiert. Die organische Phase wurde mit Wasser gewaschen, über Natriumsulfat getrocknet und eingeengt. Man erhielt 28 g Rohprodukt, das über eine Widmerkolonne destilliert wurde (Diffusionspumpe). Man erhielt 20 g (69 %) 13-Methyloxacyclopentadec-10-en-2-on, *Z*/*E* = 94/6.
IR (Film): 3008; 2927; 2857; 1734; 1459; 1378; 1245; 1180; 1146; 1055.
$^1$H-NMR ($CDCl_3$, 200 Mhz) 5.45 (2H) m; 4.2 (2H) m; 0.95 (3H) d *J* = 6.2 Hz.
$^{13}$C-NMR ($CDCl_3$) 173.9 (s); 131.3 (d); 127.8 (d); 61.9 (t); 36.2 (t); 34.0 (t); 33.9 (t); 31.2 (d); 27.62 (t); 27.60 (t); 27.1 (t); 26.8 (t); 25.6 (t); 24.8 (t) 18.6 (9).
MS 238 (2); 196 (2); 150 (5); 136 (5); 123 (5); 109 (14); 95 (30); 81 (100); 67 (49); 55 (53); 41 (48); 27 (15).
Geruch: moschusartig, fettig, fruchtig, pudrig, lactonartig.

Beispiel 5: Riechstoffkompositionen

a) Akkord: Frisch, blumig, Rose, Veilchen, z.B. für Eau de Cologne féminine geeignet

| | Gewichtsteile |
|---|---|
| Verbindung Beispiel 4 | 10 |
| BENZYLACETAT EXTRA | 40 |
| 3-CIS-HEXENYLACETAT | 1 |
| PHENYLETHYL ALKOHOL | 80 |
| α-HEXYLZIMTALDEHYD | 100 |
| BERGAMOTTE RECONSTITUTION | 150 |
| BERRYFLOR | 30 |
| CITRONELLOL EXTRA | 40 |
| CYCLAL C | 2 |
| β-DAMASCON 10 % DPG | 2 |
| DIPROPYLENGLYCOL | 90 |
| EBANOL | 5 |
| ETHYLLINALOOL | 100 |
| FLORHYDRAL | 6 |
| CITRONELLYLFORMIAT | 7 |
| GARDENOL | 4 |
| GIVESCONE | 8 |
| HEDIONE | 50 |
| HYDROXY CITRONELLAL | 30 |
| INDOLEN 10 % DPG | 5 |
| ISORALDEIN 95 | 80 |
| CIS-JASMON | 2 |
| KEPHALIS | 50 |
| LILIAL | 50 |
| ROSENOXYD | 1 |
| HEXYLSALICYLAT | 5 |
| TANGERINOL | 2 |
| TERPINEOL PUR | 30 |
| TROPIONAL | 20 |
| | 1000 |

In dieser Komposition erbringt die Verbindung des Beispiels 4 Volumen und Moschusgeruch, rundet die blumigen Noten ab, und gibt dem Akkord mehr kosmetischen Charakter.

b) Akkord: Blumig, grün, würzig, z.B. für Shampoos, Seifen, Toilettenartikel geeignet

| | Gewichtsteile |
|---|---|
| Verbindung Beispiel 4 | 15 |
| BENZYLACETAT EXTRA | 60 |
| DIMETHYLBENZYLCARBINOLACETAT | 30 |
| GERANYLACETAT | 40 |
| PHENYLETHYLALKOHOL | 120 |
| α-HEXYLZIMTALDEHYD | 120 |
| 10-UNDECEN-1-AL | 5 |
| PHENYLACETALDEHYD 85 % IN PHENYLETHYLALKOHOL | 2 |
| BERGAMOTTE GIVCO 104 | 140 |
| ZEDERNHOLZÖL VIRGIN. | 10 |
| CYCLOHEXAL | 40 |

(fortgesetzt)

| | Gewichtsteile |
|---|---|
| GERANIOL PUR | 50 |
| NELKENKNOSPENESS. | 5 |
| HEDIONE | 40 |
| HELIOTROPIN | 10 |
| ISOEUGENOL | 2 |
| ISORALDEINE 95 | 50 |
| LILIAL | 50 |
| LINALOOL SYNT. | 60 |
| MANDARINÖL COMMUNELLE | 20 |
| PECHE PURE | 1 |
| BENZYLSALICYLAT | 80 |
| 3-CIS- HEXENYLSALICYLAT | 10 |
| TROPIONAL | 10 |
| VERTOFIX COEUR | 30 |
| | 1000 |

Die Verbindung des Beispiels 4 verleiht der Komposition durch ihren moschus- und lactonartigen Charakter Volumen, rundet die grünen Noten ab und verbindet die würzigen Noten mit den blumigen Noten. Die Verbindung des Beispiels 4 erhöht zudem die Substantivität der Komposition.

## Patentansprüche

1. Riechstoffkomposition, **gekennzeichnet durch** einen Gehalt an 13-Methyloxacyclopentadec-10-en-2-on.

2. Riechstotfkomposition **gekennzeichnet durch** einen Gehalt an R-Z-13-Methyl-oxacyclopentadec-10-en-2-on.

3. Riechstoffkomposition **gekennzeichnet durch** einen Gehalt an S-Z-13-Methyloxacyclopentadec-10-en-2-on.

4. 13-Methyl-oxacyclopentadec-10-en-2-on.

5. R-Z-13-Methyl-oxacyclopentadec-10-en-2-on.

6. S-Z-13-Methyl-oxacyclopentadec-10-en-2-on.

## Claims

1. A perfume composition **characterised by** a content of 13-methyl-oxacyclopentadec-10-en-2-one.

2. A perfume composition **characterised by** a content of R-Z-13-methyl-oxacyclopentadec-10-en-2-one.

3. A perfume composition **characterised by** a content of S-Z-13-methyl-oxacyclopentadec-10-en-2-one.

4. 13-methyl-oxacyclopentadec-10-en-2-one.

5. R-Z-13-methyl-oxacyclopentadec-10-en-2-one.

6. S-Z-13-methyl-oxacyclopentadec-10-en-2-one.

## Revendications

1. Composition de parfum, **caractérisée par** une teneur en 13-méthyloxacyclopentadec-10-ène-2-one.

2. Composition de parfum, **caractérisée par** une teneur en R-Z-13-méthyloxacyclopentadec-10-ène-2-one.

3. Composition de parfum, **caractérisée par** une teneur en S-Z-13-méthyloxacyclopentadec-10-ène-2-one.

4. 13-méthyloxacyclopentadec-10-ène-2-one.

5. R-Z-13-méthyloxacyclopentadec-10-ène-2-one.

6. S-Z-13-méthyl-oxacyclopentadec-10-ène-2-one.

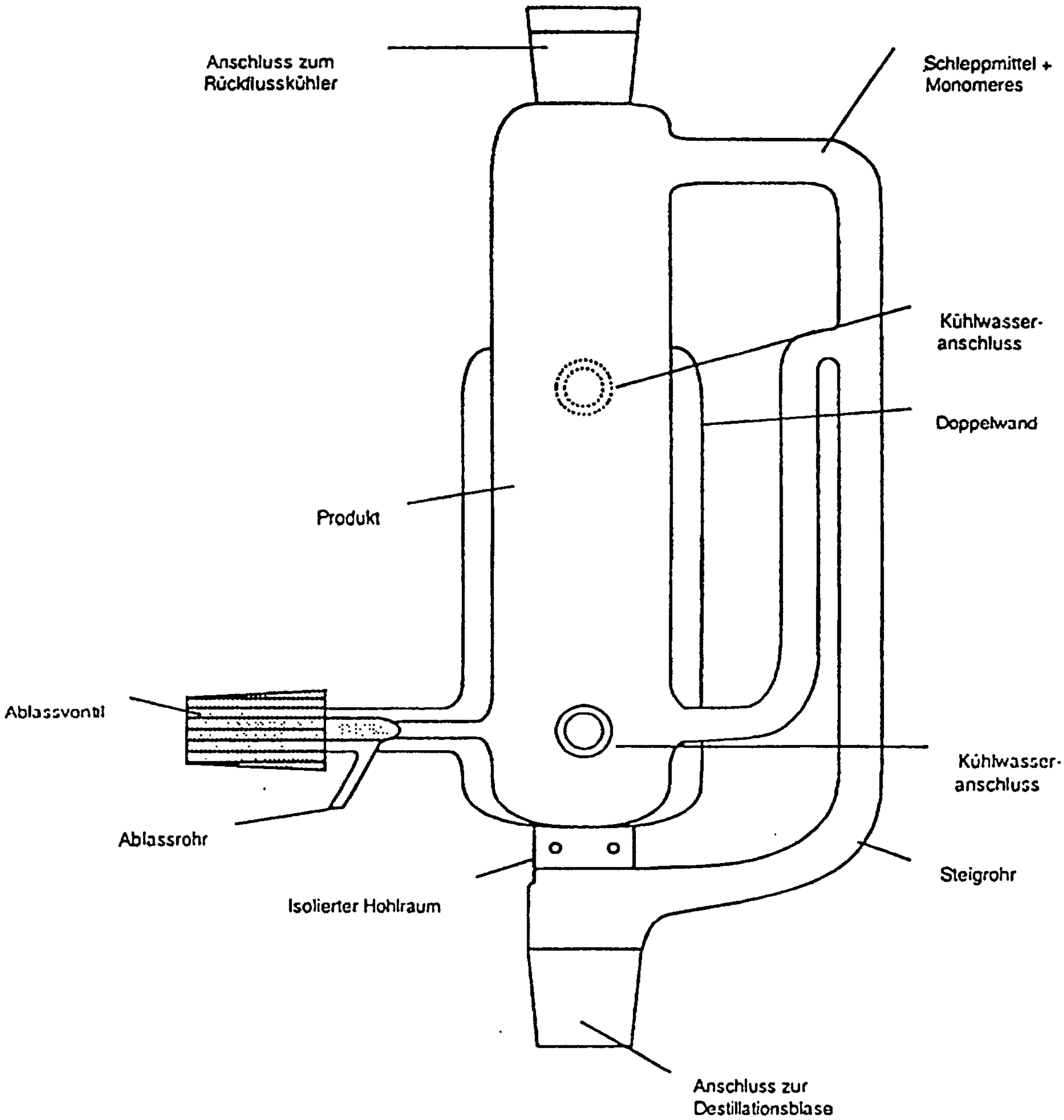
Anschluss zum
Rückflusskühler
Schleppmittel +
Monomeres
Kühlwasser-
anschluss
Doppelwand
Produkt
Ablassventil
Kühlwasser-
anschluss
Ablassrohr
Steigrohr
Isolierter Hohlraum
Anschluss zur
Destillationsblase

Figur 1